# EUROPÄISCHE PATENTANMELDUNG

(11) **EP 4 282 512 A1**
(43) Veröffentlichungstag der Anmeldung: **29.11.2023**
(21) Anmeldenummer: 23169963.8
(22) Anmeldetag: 26.04.2023
(51) Int. Cl.: B01D 53/44, A61L 9/01, B01D 53/77, F24F 3/16, F24F 8/15, F24F 8/117

(54) **LUFTSCHADSTOFFNEUTRALISATIONSREINIGER MIT POTENTIELLER DUFTFUNKTION ZUR VERBESSERUNG DER RAUMLUFT**

(30) Priorität: 27.05.2022 DE 202022102951 U
(71) Anmelder: Neutec Chemie GmbH, 55294 Bodenheim (DE); Linden Chemie GmbH & Co.KG, 53925 Kall (DE)
(72) Erfinder: Wedell, Ronni, 55294 Bodenheim (DE); Vossen, Jürgen, 53925 Kall (DE)
(74) Vertreter: Mackert, Andreas

(57) **Zusammenfassung**

Die Erfindung betrifft einen Luftschadstoffneutralisationsreiniger (Air Cleaner) zur Absorption von Schadstoffen und Geruchsreduzierung und so zur Verbesserung der Raumluft, umfassend einen Aminosäurenkomplex zumindest umfassend Bio-L-Arginin, L-Lysin und L-Prolin, wobei die Gesamtrezeptur ausschließlich natürliche Inhaltsstoffe aufweist.

## Beschreibung

Die Erfindung betrifft einen Luftschadstoffneutralisationsreiniger (Air Cleaner), der Schadstoffe absorbiert und Gerüche reduziert und so zur Verbesserung der Raumluft beiträgt.

Innenraumbelastungen mit Schadstoffen sind in zunehmendem Maße für Befindlichkeitsstörungen von Menschen verantwortlich, wobei chronische Belastungen und Kombinationswirkungen von Schadstoffen im Niedrigdosisbereich hierbei im Vordergrund stehen.

Insbesondere Formaldehydbelastungen und Aldehyde durch Zigarettenrauch, Farben, Lacke, Kleber, Desinfektionsmittel, heiße Fette u.a. belasten die Raumluft und somit häufig auch die Gesundheit der in diesen Räumen lebenden oder arbeitenden Menschen. Hinzu kommt, dass auch Möbel und sonstige Einrichtungsgegenstände sowie Bodenbeläge Schadstoffe kontinuierlich an die umgebende Raumluft abgeben können.

Zu den schwerflüchtigen organischen Verbindungen gehören neben Pestiziden und Holzschutzmitteln, die in Innenräumen überwiegend aus Quellen im Raum selbst stammen, Dioxine, Furane, polycyclische aromatische Kohlenwasserstoffe (PAK, PAH) und deren Derivate, die in Innenräumen und in der Außenluft vorkommen.

Isocyanate sind organische Verbindungen die mit Phenolen und Alkoholen zu Polyurethanen reagieren. Sie finden sich in Dispersionen, Zweikomponenten- und anderen Klebern auf Lacken und Schaumstoffen Verwendung. Die Reaktion läuft jedoch nicht vollständig ab, so dass Restmonomere im fertigen Produkt enthalten sind, die an die Innenraumluft abgegeben werden.

Vor diesem Hintergrund werden Anstrengungen unternommen, die Raumluft von dieser Vielzahl von Belastungen zu reinigen und somit gesundheitlichen Problemen der Menschen vorzubeugen, die sich in den industrialisierten Ländern Europas etwa 80% bis 90% des Tages in Innenräumen aufhalten und somit den Schadstoffen ausgesetzt sind. Gleichzeitig wird bei Einsatz von Duftstoffen auf Basis von ausschließlich natürlichen oder ätherischen Ölen die Raumluft mit angenehmem Geruch auf natürlich Weise angereichert.

Die DE 102 12 983 C1 offenbart beispielsweise einen Sanierungsbaustoff für schadstoffbelastete Gebäude bestehend aus einer Trockenmasse aus Tonmehl und Sand, dem Schafwollfasern zugesetzt sind, deren reaktive Fläche durch Schneiden kurzer Faserlängen und Aufbrechen einer oder mehrerer Faserschichten, insbesondere der Keratinschicht, vergrößert worden ist, wobei die Schafswollfasern Schadstoffe aus der Raumluft binden sollen.

Aus der Veröffentlichung US 5 705 537 A ist als anderer Baustoff ein Phenolharzschaum bekannt, der als Isolierung verwendet werden kann. Es handelt sich hier um ein geschäumtes Phenol-Formaldehyd-Resol-Harz, das ein Peptid, ein proteinhaltiges Material, Cystein, Glutaminsäure, Glycin, Isoleucin, Leucin, Lysin, Phenylalanin, Serin, Tryptophan, oder Mischungen davon in einer Menge enthält, die wirksam eine Reduzierung von Emissionen von freiem Formaldehyd aus dem geschäumten Harz bewirken.

In der offenbarten Erfindung der CA 1 162 904 A wird ein Verfahren der Chemiesorption eingesetzt, um gasförmiges Formaldehyd zu festigen, in nicht flüchtigen Verbindungen umzuwandeln und in den Absorber zu binden. Der Absorber kann hierfür in verschiedenen Formen von Filtern hergestellt werden, und eignet sich für beruflichen Atemschutz, Luft-Reinigung in Arbeits- und Wohnbereichen und für die Formaldehyd-Absorption von Prozessgasen. Der Absorber kann hier auch präventiv in Kunstharzen zur Reduzierung von Formaldehyd-Emissionen integriert werden.

Dies berücksichtigend ist es die Aufgabe der vorliegenden Erfindung, einen Luftschadstoffneutralisationsreiniger (Air Cleaner) zu schaffen, der eine konstante Neutralisierung von Gerüchen in der Raumluft ermöglicht und hierbei Schadstoffe wie Formaldehyde und Aldehyde und andere Schadstoffe katalysiert. Der Luftschadstoffneutralisationsreiniger soll hierbei sehr absorptionsfähig und langfristig wirksam, gleichzeitig aber auch einfach und ungefährlich in der Anwendung sein. Zudem sollen möglichst hohe Standards in Bezug auf natürliche Inhaltsstoffe und somit die ökologische Verträglichkeit des Produktes verwirklicht werden.

Die Aufgabe wird gelöst durch den erfindungsgemäßen Luftschadstoffneutralisationsreiniger (Air Cleaner) mit den Merkmalen des Anspruchs 1.

Die Unteransprüche haben vorteilhafte Weiterbildungen des Luftschadstoffneutralisationsreinigers (Air Cleaners) zum Gegenstand.

Der erfindungsgemäße Luftschadstoffneutralisationsreiniger (Air Cleaner) verwirklicht die Aufgabenstellung zum einen durch einen neuartigen Aminosäurenkomplex, wodurch mit erhöhter Wirksamkeit Schadstoffe absorbiert und Gerüche reduziert werden und so eine Verbesserung der Raumluft erfolgt.

Es wurde durch experimentelle Versuchsreihen ein erfindungsgemäßer Aminosäurenkomplex als besonders vorteilhaft festgestellt, um diese Aufgabenstellung als Bestandteil eines Luftreinigers zu verwirklichen, wobei dieser Aminosäurenkomplex zumindest Bio-L-Arginin, L-Lysin und L-Prolin, umfasst und die Gesamtrezeptur ausschließlich natürliche Inhaltsstoffe aufweist.

Dieser im erfindungsgemäßen Luftschadstoffneutralisationsreiniger enthaltene Aminosäurenkomplex in der offenbarten Wirkstoffkombination, weist aufgrund der Wechselwirkung bzw. dem Zusammenwirken der offenbarten Bestandteile gegenüber bekannten Verbindungen veränderte physikalisch-chemische Parameter und daraus resultierend veränderte biologische Eigenschaften bzw. Wirksamkeiten auf, wodurch sich gezeigt hat, dass dieser Aminosäurenkomplex die angestrebte Aufgabenstellung bei der Schadstoffneutralisation besser erfüllen kann. Der erfindungsgemäße Luftschadstoffneutralisationsreiniger ist hierdurch in der Lage, mit den Luftschadstoffen in verbesserter Form chemisch zu reagieren und diese wirksam zu neutralisieren. Zudem werden unangenehme Geruchsstoffe in ihrer Wirkung vermindert bzw. durch enthaltene Duftstoffe überlagert.

Bevorzugte Ausführungsformen der erfindungsgemäßen Zusammensetzung sind definiert durch Anteil-Unter- und Obergrenzen in der nachfolgenden Tabelle A dargestellt:

**Tabelle A**

| **Inhaltsstoff** | **Anteils-Untergrenze** | **Anteils-Obergrenze** |
|---|---|---|
| Wasser | 70,00% | 90,00% |
| Bio-Ethanol | 6,00% | 15,00% |
| Bio-L-Arginin | 0,50% | 3,00% |
| L-Lysin | 0,30% | 2,00% |
| L-Prolin | 0,04% | 1,00% |
| Polyglyceryl | 2,00% | 5,00% |
| Zitronensäure | 0,20% | 1,00% |
| | **Summe: 100,00%** | |

Bei der Formulierung des erfindungsgemäßen Luftschadstoffneutralisationsreinigers unter Berücksichtigung der Problemstellung, eine hohe Wirksamkeit in Bezug auf die Neutralisierung von Gerüchen und Schadstoffen in der Raumluft zu erreichen und gleichzeitig möglichst hohe Standards in Bezug auf natürliche Inhaltsstoffe und somit die ökologische Verträglichkeit des Produktes zu verwirklichen, ergaben sich zu überwindende Herausforderungen, eine phasenstabile und klargestellte Mischung zu erreichen.

Unter einer phasenstabilen oder phasenfreien Verbindung im Sinne der Erfindung ist zu verstehen, dass das Produkt homogen ist und es somit nur eine Phase gibt. Das bedeutet, dass es nur einen Aggregatzustand aufweist und als homogenes Gemisch einphasig vorliegt.

Dieser Zustand wurde experimentell durch den erfindungsgemäßen Luftschadstoffneutralisationsreiniger der angeführten Formulierung erreicht. Insbesondere ist hier der optionale Zusatz ätherischer Öle als Duftstoffe problematisch. Aufgrund der Aufgabenstellung, möglichst hohe Standards in Bezug auf natürliche Inhaltsstoffe zu erfüllen, sind Duftstoffe in Form ätherischer Öle als Naturprodukte zu verwenden, was die Herausforderung vergrößert, ein homogenes und phasenstabiles Produkt zu schaffen. Die angegebenen Aminosäuren in deren angegebenen Anteilen an der Mischung und in Kombination mit Polyglyceryl als Emulgator und/oder zur Regelung der Viskosität hat sich als Kombination gezeigt, die diese angestrebte phasenstabile Homogenität der Mischung der natürlichen Inhaltsstoffe bei hoher Wirksamkeit in Bezug auf die Neutralisierung von Gerüchen und Schadstoffen realisiert.

Unter der klargestellten Mischung im Sinne der vorliegenden Erfindung ist zu verstehen, dass in der als Produkt vorliegenden Flüssigkeit ein optisch trüber Eindruck vermieden wird. Eine Trübung durch kleine Partikel in der Flüssigkeit, die eine vom Trägerstoff abweichende Brechzahl besitzen, soll so vermieden werden, da dies als Anwendererwartung für den vorliegenden Luftschadstoffneutralisationsreiniger besteht und ein trübes Produkt weniger Akzeptanz erfährt.

Auch für diese Anforderung an das Produkt hat die Auswahl der angegebenen Aminosäuren als Aminosäuren-Komplex in deren angegebenen Anteilen an der Mischung in Kombination mit Polyglyceryl gute Ergebnisse erzielt. Die Mischung konnte auch bei der angestrebten Verwendung der rein natürlichen Inhaltsstoffe klargestellt werden, ohne Einbußen in Bezug auf eine hohe Wirksamkeit in Bezug auf die Neutralisierung von Gerüchen und Schadstoffen in Kauf nehmen zu müssen.

Eine andere durch die Verwendung der natürlichen Inhaltsstoffe zu überwindende Problematik im Bereich des Aminosäuren-Komplexes ist der pH-Wert, der für die angestrebt hohe Wirksamkeit in Bezug auf die Neutralisierung von Gerüchen und Schadstoffen in der Raumluft tendenziell im neutralen Bereich liegen sollte. Das Arginin im erfindungsgemäßen Luftschadstoffneutralisationsreiniger wird in Form von Bio-L-Arginin verwendet. Im Stand der Technik wird Arginin in beispielsweise Reinigungslösungen als sogenanntes HCL-Produkt eingesetzt, wobei hier der Arginin-Gehalt deutlich geringer ist aber annähernd neutral verliegt und die Aminosäure im Salz der Salzsäure (HCL) gebunden ist.

Das erfindungsgemäß verwendete Bio-L-Arginin zählt zu den basischen Aminosäuren mit nahezu 100 % der Aminosäure und insofern einer deutlich höheren Konzentration und Reinheit der Aminosäure, was sich als positiv für die angestrebt hohe Wirksamkeit in Bezug auf die Neutralisierung von Gerüchen erwiesen hat. Die Bio-L-Arginin Aminosäure liegt erfindungsgemäß in Food-Grade-Qualität vor, was das Produkt zusätzlich in Bezug auf Gesundheits- und Umweltverträglichkeit hin verbessert.

Im Gegensatz zu dem im Stand der Technik verwendeten Arginin in HCL Qualität und mit einem in etwa neutralen PH-Wert weist Bio-L-Arginin einen basischen pH-Wert von etwa 11 auf, wobei es für die Wirksamkeit der Aminosäuren als Schadstoffbinder oder -neutralisierer zentral ist, dass das Produkt in etwa einen neutralen pH-Wert von 7 aufweist. Es hat sich hierbei als vorteilhaft herausgestellt, in die vorliegende Formulierung den Inhaltsstoff der Zitronensäure aufzunehmen, die als pH-Wert-Einsteller verwendet wird, um so den pH-Wert auf den neutralen Wert von etwa 7 einzustellen. Auch dies ist demnach ein wichtiger Aspekt der Formulierung, um ECOzertifizierte Inhaltsstoffe mit hoher Wirksamkeit in einem Produkt zusammenzuführen.

Die Mengenverhältnisse des Aminosäurenkomplexes mit den Verhältnissen der Aminosäuren zueinander wie auch das Verhältnis des Polyglyceryls als Emulgator und/oder zur Regelung der Viskosität zum Aminosäurenkomplex haben sich als besonders positiv erwiesen, um eine hohe Wirksamkeit mit einer optimalen Umweltverträglichkeit in Einklang zu bringen. Des Weiteren hat sich das Mengenverhältnis der Zitronensäure in der Mischung gegenüber dem Aminosäurenkomplex als ideal gezeigt, um über den neutralen pH-Wert die Wirksamkeit zusätzlich zu verbessern.

Die in Tabelle A angegebenen Inhaltsstoffe sind hierbei nicht abschließend zu verstehen, da weitere Inhaltsstoffe zugesetzt werden können. Dies betrifft beispielsweise Duftstoffe in Form natürlicher und/oder ätherischer Öle, auf die bereits eingegangen wurde. Bei den angegebenen anteiligen Mengenangaben wird dies je nach Duftstoffmengenanteil beispielsweise zu einer Reduzierung des Wasseranteils führen. Auch die weiteren Anteile der Inhaltsstoffe können sich hierdurch geringfügig verändern.

Der erfindungsgemäße Luftschadstoffneutralisationsreiniger (Air Cleaner) nach Anspruch 1 unterscheidet sich grundsätzlich sowohl im Einsatzprofil als auch im Wirkungsspektrum von herkömmlichen Luftreinigern. Es ist für den Abbau von Reiz- und Geruchsstoffen in der Innenraumluft durch Chemieabsorption optimiert und enthält synergistisch wirkende Komponenten eines Aminosäurenkomplexes, das als Absorptionsmittel insbesondere für gasförmige, proteinreaktive Substanzen in Innenräumen zum Einsatz kommt. Hierzu gehören u.a. Aldehyde und VOC (volatile organic compounds = flüchtige organische Verbindungen).

Der Luftschadstoffneutralisationsreiniger (Air Cleaner) dient so der Aufnahme und Abreaktion von Reiz- und Geruchstoffen belasteter Innenräume und der Prävention derartiger Belastungen.

Die wesentlichen Verbesserungen, die durch den erfindungsgemäßen Luftschadstoffneutralisationsreiniger erzielt werden, sind nachfolgend nicht abschließend und beispielhaft angeführt.
- Abbau von unter anderem Formaldehydverbindungen.
- Das Vermeiden externer Quellen wie Luftfilter, beispielsweise Schwebstofffilter wie HEPA-Filter (HEPA = High-Efficiency Particulate Air/Arrestance), Aktivkohlefilter oder UV-C-Luftreiniger- oder UV-Luftdesinfektionsgeräte, wie auch Licht, Sauerstoff oder Ionentauscher, mit denen die Luft gemäß zuvor beschriebener Anwendung gereinigt wird, wird erreicht.
- Eine Mehr-Stunden-Funktionalität bzw. Langzeitwirkung wird bei einer Kombination mit beispielsweise Lufterfrischern oder Luftwäschern ermöglicht.
- Der Abbau und die Neutralisation von insbesondere proteinreaktiven Reiz- und Geruchstoffen konnte nachgewiesen werden.
- Reaktions- und Reaktionszwischenprodukte wurden experimentell als unbedenklich identifiziert.
- Es werden 100 % natürliche Rohstoffe verwendet, deren biologische Abbaubarkeit nachgewiesen ist. Somit erfolgt eine Zertifizierung nach dem hohen ECOCERT Standard "Kerzen und Raumdüfte".
- Es erfolgt die Anwendung des neuartigen Aminosäurewirkstoffkomplexes, der in Kombination mit den weiteren Inhaltsstoffen zum einen die hohe Wirksamkeit und zum anderen den hohen ECOCERT Standard ermöglicht.

Der erfindungsgemäße Luftschadstoffneutralisationsreiniger (Air Cleaner) bewirkt somit nachweislich positive Auswirkungen in Bezug auf ein verbessertes Raumklima durch Abbau von Geruchs- und Schadstoffen bei gleichzeitig deutlich verbesserter Umweltverträglichkeit.

Technisch neu im Vergleich zu bestehenden Luftreinigungs-Lösungen, beispielsweise durch Aktivkohlefilter, ist zudem, dass Reiz-, Schad- und Geruchsstoffe chemisch umgesetzt, dauerhaft gebunden und im Fall von proteinreaktiven Verbindungen neutralisiert werden unter Vermeidung von Geruchs- bzw. Schadstoff Überdeckungen.

Der Einsatz eines mit ECOCERT zertifizierten Rohstoffen und einem derartig zusammengesetzten Aminosäurenkomplexes in einem Luftreiniger ist bisher im Stand der Technik nicht offenbart oder nahegelegt. Zudem unterliegt die Zertifizierung unter dem ECOCERT "Kerzen und Raumdüfte" Standard höchsten Ansprüchen an die 100 % natürlichen Inhaltsstoffe und ebenso an die Nachhaltigkeit des gesamten Produktes. Dieser Standard stellt die höchsten Ansprüche an die natürliche Herkunft aller Rohstoffe des Produktes und einen nachgewiesenen biotechnologischen Herstellungsprozess jedes einzelnen Rohstoffes. Gentechnische Veränderungen sind in diesem Standard vollumfänglich ausgeschlossen.

## Patentansprüche

1. Luftschadstoffneutralisationsreiniger (Air Cleaner) zur Absorption von Schadstoffen und Geruchsreduzierung und so zur Verbesserung der Raumluft,
**gekennzeichnet durch**
- einen Aminosäurenkomplex zumindest umfassend Bio-L-Arginin, L-Lysin und L-Prolin,
- wobei die Gesamtrezeptur ausschließlich natürliche Inhaltsstoffe aufweist.

2. Luftschadstoffneutralisationsreiniger (Air Cleaner) nach Anspruch 1,
**dadurch gekennzeichnet, dass**
als Emulgator und/oder zur Regelung der Viskosität Polyglyceryl anteilig in der Rezeptur vorhanden ist.

3. Luftschadstoffneutralisationsreiniger (Air Cleaner) nach Anspruch 1 oder 2,
**dadurch gekennzeichnet, dass**
als pH-Wert-regulierender Inhaltsstoff Zitronensäure anteilig in der Rezeptur vorhanden ist.

4. Luftschadstoffneutralisationsreiniger (Air Cleaner) nach einem der vorangegangenen Ansprüche,
**dadurch gekennzeichnet, dass** die Rezeptur zumindest umfasst:
- 70,00% bis 90,00% Wasser,
- 6,00% bis 15,00% Bio-Ethanol,
- 0,50% bis 3,00% Bio-L-Arginin,
- 0,30% bis 2,00% L-Lysin,
- 0,04% bis 1,00% L-Prolin,
- 2,00% bis 5,00% Polyglyceryl sowie
- 0,20% bis 1,00% Zitronensäure.

5. Luftschadstoffneutralisationsreiniger (Air Cleaner) nach einem der vorangegangenen Ansprüche,
**dadurch gekennzeichnet, dass**
als Duftstoff zumindest ein natürliches und/oder ätherisches Öl anteilig in der Rezeptur vorhanden ist.

6. Luftschadstoffneutralisationsreiniger (Air Cleaner) nach einem der vorangegangenen Ansprüche,
**dadurch gekennzeichnet, dass**
in der Rezeptur 100% ECOCERT-zertifizierte Inhaltsstoffe des Kerzen-und-Raumdüfte-Standards für ökologische Kerzen und Raumdüfte natürlichen Ursprungs enthalten sind.
